# EUROPÄISCHE PATENTSCHRIFT

(11) **EP 1 486 228 B1**
(45) Veröffentlichungstag und Bekanntmachung des Hinweises auf die Patenterteilung: **26.03.2008**
(21) Anmeldenummer: 04013757.2
(22) Anmeldetag: 11.06.2004
(51) Int. Cl.: A61M 16/04

(54) **Tracheostomaventil**
Tracheostomy valve
Valve de trachéotomie

(30) Priorität: 12.06.2003 DE 10326829
(43) Veröffentlichungstag der Anmeldung: 15.12.2004
(73) Patentinhaber: ESKA-medical Gesellschaft für Entwicklung und Vertrieb von medizinischen Implantat-Artikeln mbH, 23556 Lübeck (DE)
(72) Erfinder: Grundei, Hand, Dr., 23558 Lübeck (DE)
(74) Vertreter: Fuchs

(56) Entgegenhaltungen:
- EP-A- 0 221 973
- WO-A-91/07202
- US-A- 4 649 913
- US-B1- 6 358 222

## Beschreibung

Die vorliegende Erfindung betrifft ein Tracheostomaventil. Derartige Ventile sind bekannt beispielsweise aus der EP-B-0221973 oder aus der EP-B-0617630. Ein derartiges Tracheostomaventil weist einen Ventilkörper auf, der auf einem Kanülenstummel sitzt bzw. in diesen integriert ist, welcher in ein Tracheostoma setzbar ist und ein in die Trachea einbringbares Abschlussorgan aufweist. Ein anderer Typus eines Tracheostomaventils weist ebenfalls einen Ventilkörper auf, der jedoch auf einer Tracheostomiekanüle sitzt bzw. in dieser integriert ist, welche in ein Tracheostoma setzbar ist. Während der erstgenannte Typ für den Einsatz eines Sprechventiles, welches in einen künstlichen, zwischen Trachea und Oesophagus eingebrachten Shunt setzbar ist, konzipiert ist, ist dies beim zweitgenannten Typ nicht der Fall, das heißt, dieses Tracheostomaventil kommt zum Einsatz ohne dass ein Sprechventil angewendet wird.

Bei dem beispielsweise in der letztgenannten Druckschrift genannten Tracheostomaventil handelt es sich um ein solches mit einem Ventilschließglied, das unter Ansprechen auf eine Luftverschiebung das Ventil schließt. Dies wird vom Patienten veranlasst, um die Luft durch ein Shunt-Ventil zwischen Trachea und Oesophagus leiten zu können, um so eine gewisse Sprechmöglichkeit aufrechtzuerhalten. Das erwähnte Ventil weist darüber hinaus ein so genanntes Ablassventilglied auf, das sich unter Ansprechen auf einen besonderen Überdruck auf der Trachea-Seite des Ventils öffnet. Beim Husten ist somit dafür Sorge getragen, dass sich das Ablassventil öffnet und die Luft und Sputum aus dem Stoma herausgepresst werden kann. Diese Funktion ist wichtig, da es ansonsten zu schweren Komplikationen kommen kann, wenn beispielsweise das Sekret (Sputum) in die Lungenflügel gelangt, wo es zu schweren Infektionen führen kann.

Vorraussetzung für die Funktionsfähigkeit des Tracheostomaventils ist der dichte Sitz des Ventils im Stoma. In einigen Fällen hat sich gezeigt, dass im unteren Bereich des Tracheostomas eine Einwölbung oder Einsenkung entsteht, welche die Abdichtung erschwert. Hierzu ist in der erstgenannten Druckschrift vorgesehen, einen Flanschring auf einem in dem Tracheostoma sitzenden Kanülenstummel anzuformen, der das Stoma vollständig abdichten soll.

Die Anwendung eines Tracheostomaventils brachte enorme Fortschritte gegenüber den zuvor verwendeten Membranventilen. Ein derartiges Membranventil wurde auf dem Stoma angebracht, und zwar unter Verwendung einer Klebefolie. Dabei treten jedoch erheblich Schwierigkeiten hinsichtlich der Haltbarkeit und Abdichtung auf, ganz abgesehen von den Problemen, die mit der aufgrund der Tumorerkrankung durch Bestrahlung gereizten Haut einhergehen, wenn sie mit einer Klebefolie versehen werden soll.

Vor diesem Hintergrund ist es die Aufgabe der vorliegenden Erfindung, Tracheostomaventile der eingangs genannten Art beiderlei Typen so weiterzubilden, dass die Abdichtung des Stomas sicher bewerkstelligt werden kann und dass der Tragekomfort für den Patienten erhöht wird.

Gelöst wird diese Aufgabe durch ein Tracheostomaventil des ersten Typs mit den Merkmalen des Anspruchs 1. Vorteilhafte Weiterbildungen sind in den Unteransprüchen 2 bis 11 angegeben.

Demnach werden wenigstens zwei in Richtung der Hauptachse des Kanülenstummels axial versetzte, auf dem Kanülenstummel umlaufende, ringförmige Dichtlamellen vorgeschlagen, die sich nach Einsetzen des Kanülenstummels in das Tracheostoma an die das Tracheostoma umgebende Haut in dichtender Weise anlegen.

Mehrere Lamellen legen sich wie ein Lamellenvorhang um den Rand des Tracheostomas. Dies erhöht den Tragekomfort erheblich. Da nicht nur eine Lammelle als Dichtung vorgesehen ist, sondern mehrere, wird der Abdichteffekt durch die Hintereinanderschaltung mehrerer Dichtungen deutlich erhöht, sodass die Funktionsweise der in den erwähnten Druckschriften beschriebenen Tracheostomaventile gewährleistet ist. Vorteilhaft sind drei Dichtlamellen vorgesehen.

In bevorzugter Ausführung sind die Dichtlamellen in Richtung auf das Abschlussorgan konkav ausgebildet. Dies führt zu einer weiteren Erhöhung der Abdichtwirkung aufgrund der wirkenden Rückstellkräfte, die entstehen, wenn das Tracheostomaventil in dem Stoma sitzt.

Bevorzugt ist, wenn die Außendurchmesser der Dichtlamellen von innen in Richtung des Ventilkörpers stetig zunehmen. Hierdurch wird gewissermaßen eine Kaskade von Dichtungen erzeugt. Die innerste Dichtlammelle, also die innen liegende, hat den kleinsten Durchmesser. Hierdurch legt sich die größte, äußere Lichtlamelle über die weiteren Dichtlamellen mit jeweils kleinerem Durchmesser. Der Durchmesser der kleinsten Dichtlamelle muss hierbei größer sein als das Stoma selbst. Bildlich gesprochen wird so eine Kaskade von übereinander liegenden abdichtenden Glocken erzeugt. Dies gewährleistet die Abdichtung des Stomas auch bei Bewegungen des Patienten, etwa beim Heben oder Senken des Kopfes.

Bevorzugt wird, wenn die Dichtlamellen an dem Kanülenstummel direkt angeformt sind und vorzugsweise aus demselben Material wie Silikongummi bestehen. Dann kann dieses Anschlussteil als Ganzes in einer Form hergestellt werden.

Das erwähnte Abschlussorgan ist bevorzugt ein an das innere Ende des Kanülenstummels angesetztes, zum Kanülenstummel offenes Rohrstück. Dies setzt voraus, dass die Trachea nicht in der früher üblichen Weise unter Biegung zum Stoma geführt ist, sondern dass die Trachea nach Absetzen des Kehlkopfes einen Deckel erhält und das Stoma seitlich in die Trachea geführt ist. Dadurch wird auch ein großlumiges Stoma mit einem entsprechend großen Querschnitt des Verschlusses ermöglicht.

Mit Vorteil weist das Rohrstück an der dem Kanülenstummel gegenüberliegenden Wand eine Öffnung auf zum Einsetzen einer Stimmprothese in den Shunt zwischen Trachea und Oesophagus.

Eine andere vorteilhafte Weiterbildung ist darin zu sehen, wenn das Rohrstück auf der dem Kanülenstummel gegenüberliegenden Seite vollständig geöffnet ist, sodass ein rinnenförmiges Gebilde entsteht.

Besonders bevorzugt wird eine Ausführungsform, bei der an den Enden des Rohrstückes jeweils ein Flansch aus biegsamem Material angeformt ist, das in sich in Richtung Kanülenstummel gebogen ist. Diese beiden Flansche legen sich gegen die Tracheawand und erzeugen so eine Zugkraft, welche bestrebt ist, den Ventilkörper weiter in das Tracheostoma zu ziehen. Damit aber werden die Lamellen weiter in Richtung Stoma gezogen und die Dichtwirkung wird erhöht.

Günstig ist es, wenn der Kanülenstummel, die Dichtlamellen, das Rohrstück und die Flansche einstückig ausgebildet sind. Dies vereinfacht die Herstellung dieses Anschlussteils erheblich.

Die angegebene Aufgabe wird auch durch ein Tracheostomaventil des zweiten Typs mit den Merkmalen des Anspruchs 12 gelöst. Vorteilhafte Weiterbildungen sind in den Unteransprüchen 3 bis 17 angegeben.

Demnach werden wenigstens zwei in Richtung der Hauptachse des Einsatzstückes der Tracheostomiekanüle axial versetzte, auf dem Einsatzstück umlaufende, ringförmige Dichtlamellen vorgeschlagen, die sich nach Einsetzen der Tracheostomiekanüle in das Tracheostoma an die das Tracheostoma umgebende Haut in dichtender Weise anlegen.

Mehrere Lamellen legen sich wieder wie ein Lamellenvorhang um den Rand des Tracheostomas. Dies erhöht den Tragekomfort erheblich. Da nicht nur eine Lammelle als Dichtung vorgesehen ist, sondern mehrere, wird der Abdichteffekt durch die Hintereinanderschaltung mehrerer Dichtungen deutlich erhöht, sodass die Funktionsweise der in den erwähnten Druckschriften beschriebenen Tracheostomaventile gewährleistet ist. Vorteilhaft sind drei Dichtlamellen vorgesehen.

In bevorzugter Ausführung sind hier die Dichtlamellen in Richtung auf den Ventilkörper konvex ausgebildet. Dies führt zu einer weiteren Erhöhung der Abdichtwirkung aufgrund der wirkenden Rückstellkräfte, die entstehen, wenn das Tracheostomaventil in dem Stoma sitzt.

Bevorzugt ist auch bei diesem Typ, wenn die Außendurchmesser der Dichtlamellen von innen in Richtung des Ventilkörpers stetig zunehmen. Hierdurch wird gewissermaßen eine Kaskade von Dichtungen erzeugt. Die innerste Dichtlammelle, also die innen liegende, hat den kleinsten Durchmesser. Hierdurch legt sich die größte, äußere Lichtlamelle über die weiteren Dichtlamellen mit jeweils kleinerem Durchmesser. Der Durchmesser der kleinsten Dichtlamelle muss hierbei größer sein als das Stoma selbst. Bildlich gesprochen wird so eine Kaskade von übereinander liegenden abdichtenden Glocken erzeugt. Dies gewährleistet die Abdichtung des Stomas auch bei Bewegungen des Patienten, etwa beim Heben oder Senken des Kopfes.

Bevorzugt wird, wenn die Dichtlamellen an dem Einsatzstück der Tracheostomiekanüle direkt angeformt sind und vorzugsweise aus demselben Material wie Silikongummi bestehen. Dann kann dieses Anschlussteil als Ganzes in einer Form hergestellt werden.

Die Erfindung wird beispielhaft anhand von Ausführungsbeispielen gemäß den Zeichnungsfiguren näher erläutert. Hierbei zeigt:
- Fig. 1: einen schematischen Schnitt durch den Halsbereich eines mit dem Tracheostomaventil des ersten Typs versorgten Patienten,
- Fig.2: die Seitenansicht des Tracheostomaventils, seitlich geschnitten,
- Fig. 3: eine perspektivische Ansicht des Tracheostomaventils, von schräg hinten gesehen, und
- Fig. 4: einen schematischen Schnitt durch den Halsbereich eines mit dem Tracheostomaventil des zweiten Typs versorgten Patienten.

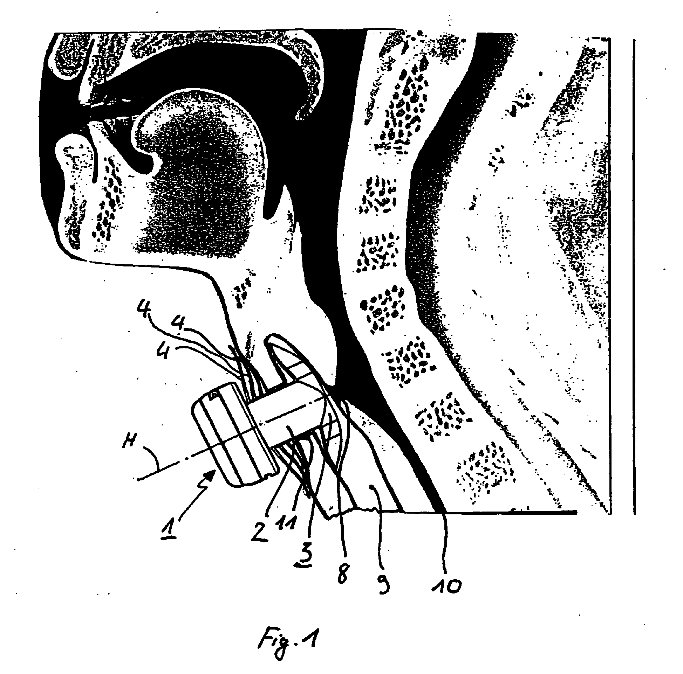

Einen ersten Überblick verschafft Fig. 1. Zwischen Trachea 9 und Oesophagus 10 hat der Operateur einen Shunt 8 gelegt, in welchen eine Stimmprothese eingesetzt werden kann. Diese ist vorliegend aus Darstellungsgründen nicht gezeigt. In ein erzeugtes Tracheostoma 11 ist das Tracheostomaventil gesetzt. Dies besteht aus dem Ventilkörper 1 und dem Kanülenstummel 2 sowie einem Abschlussorgan 3. Der Kanülenstummel 2 sitzt direkt in dem Tracheostoma 11. Das Abschlussorgan weist rückseitig eine Öffnung auf, durch welche hindurch eine Stimmprothese (nicht dargestellt) in den Shunt 8 gesetzt werden kann. Drei Dichtlamellen 4 in Form von auf dem Kanülenstummel 2 umlaufenden Kreisringen sorgen für eine sichere Abdichtung des Tracheostomas 11. Die Dichtlamellen 4 sind in Richtung der Hauptachse H des Kanülenstummels 2 axial versetzt. Darüber hinaus nehmen die Außendurchmesser der Dichtlamellen 4 in Richtung auf den Ventilkörper stetig zu. Dies bedeutet, dass die den Ventilkörper 1 am nächstgelegene Dichtlamelle 4 den größten Außendurchmesser hat und die dem Tracheostoma 11 am entferntest gelegene Dichtlamelle 4 den kleinsten Durchmesser hat. Selbst der kleinste Durchmesser der innen liegenden Dichtlamelle ist jedoch größer als der Durchmesser des Tracheostomas 11. Mit dieser Anordnung entsteht eine Kaskade von Dichtungen, die sich übereinander legen, sodass der Patient seinen Kopf bewegen, beispielsweise heben oder senken kann, ohne dass es zu Undichtigkeiten kommen würde.

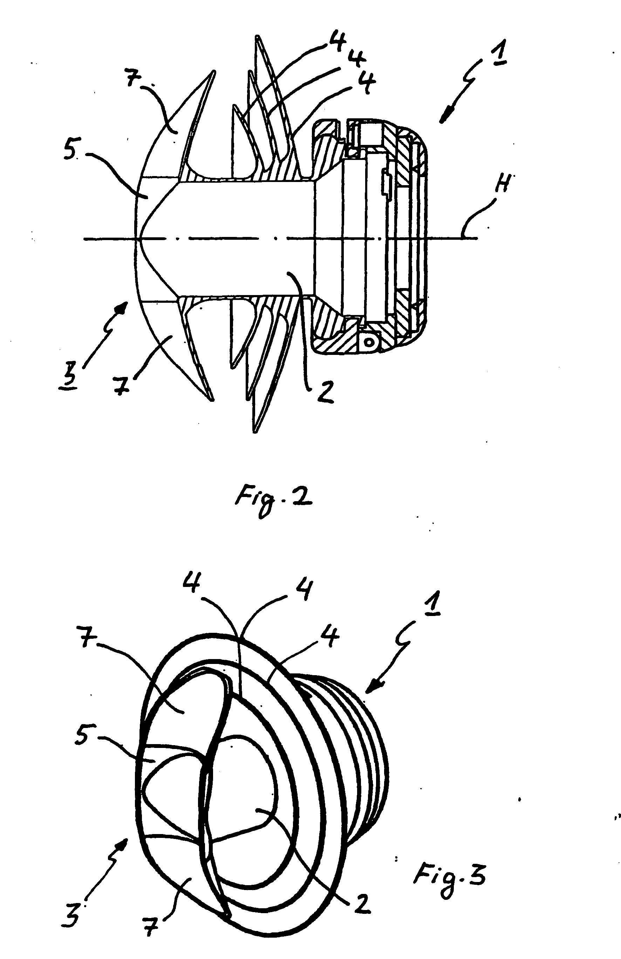

Weitere Einzelheiten ergeben sich aus der Fig. 2. Auf den Kanülenstummel 2 ist ein Ventilkörper 1 gesetzt. Dieser Ventilkörper 1 könnte beispielsweise einer gemäß der EP-B-0617630 sein. Vorliegend am Kanülenstummel 2 angeformt sind drei Dichtlamellen 4 in leicht konkav ausgeführter Form. Die dem Ventilkörper 1 nächstgelegene Dichtlamelle 4 hat den größten Durchmesser, während die mittlere Dichtlamelle einen Durchmesser aufweist, der zwischen dem größten und dem kleinsten Durchmesser der innen liegenden Dichtlamelle liegt.

Das Abschlussorgan 3 ist vorliegend als Rohrstück 5 ausgebildet, an dessen Enden jeweils ein Flansch 7 angeformt ist. Die Flansche 7 bestehen aus einem biegsamen Material, vorzugsweise aus demselben wie der Kanülenstumpf 2 und die Dichtlamellen 4 sind in Richtung des Kanülenstummels 2 gebogen. Diese Biegung der Flansche 7 ist im Zusammenhang mit der leichten konkaven Ausprägung der ringförmigen Dichtlamellen 4 zu sehen. Die Flansche 7 kommen an der Tracheawand zur Anlage und erzeugen eine Zugkraft, welche bestrebt ist, den Ventilkörper 1 weiter in das Tracheostoma zu ziehen. Die Konkavität der Dichtlamellen 4 bewirkt nun ein Ausfedern der Dichtlamellen hin zum Ventilkörper 1 unter bleibender Anlage am Hals des Patienten. Auf diese Weise ist sichergestellt, dass auch bei Bewegung des Kopfes eine sichere Abdichtung des Tracheostomas gewährleistet bleibt.

Wie Fig. 3 zeigt, ist in dem dargestellten Ausführungsbeispiel das Rohrstück 5 auf der dem Kanülenstummel 2 gegenüberliegenden Seite vollständig geöffnet. Dadurch entsteht ein rinnenförmiges Gebilde.

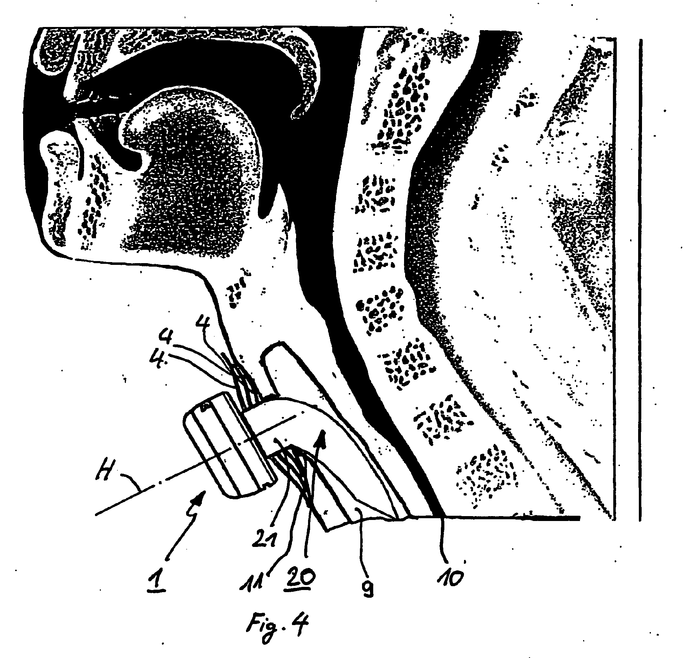

Fig. 4 veranschaulicht den Einsatz des Tracheostomaventils des zweiten Typs. Hier findet keine Versorgung des Patienten mit einem Sprechventil statt. Vielmehr kommt vorliegend eine Tracheostomiekanüle 20 mit ihrem hier zylindrischen Einsatzstück 21 zur Anwendung. Es ist kein Shunt zwischen der Trachea 9 und dem Oesophagus 10 gelegt worden. In das erzeugte Tracheostoma 11 ist wiederum das Tracheostomaventil gesetzt, welches wiederum einen Ventilkörper 1 aufweist, welcher vorliegend auf der Tracheostomiekanüle 20 sitzt.

Drei Dichtlamellen 4 in Form von auf dem Einsatzstück 21 der Tracheostomiekanüle 20 umlaufenden Kreisringen sorgen für eine sichere Abdichtung des Tracheostomas 11. Die Dichtlamellen 4 sind wieder in Richtung der Hauptachse H des Einsatzstückes 21 axial versetzt. Dabei nehmen die Außendurchmesser der Dichtlamellen 4 in Richtung auf den Ventilkörper 1 stetig zu. Die Verhältnisse der Dichtlamellen entsprechen jenen beim Tracheostomaventil des ersten Typs gemäß Fig. 1, weswegen an dieser Stelle auf die Beschreibung weiter oben verwiesen wird.

## Patentansprüche

1. Tracheostomaventil mit einem Ventilkörper (1), der auf einem Kanülenstummel (2) sitzt, bzw. in diesen integriert ist, welcher in ein Tracheostoma setzbar ist und ein in die Trachea einbringbares Abschlussorgan (3) aufweist,
**gekennzeichnet durch**,
wenigstens zwei in Richtung der Hauptachse (H) des Kanülenstummels (2) axial versetzte, auf dem Kanülenstummel (2) umlaufende ringförmige Dichtlamellen (4), die sich nach Einsetzen des Kanülenstummels (2) in das Tracheostoma (11) an die das Tracheostoma (11) umgebende Haut in dichtender Weise anlegen.

2. Tracheostomaventil nach Anspruch 1, **dadurch gekennzeichnet, dass** die Dichtlamellen (4) in Richtung auf das Abschlussorgan (3) konkav ausgebildet sind.

3. Tracheostomaventil nach Anspruch 1 oder 2, **dadurch gekennzeichnet, dass** drei Dichtlamellen (4) vorgesehen sind.

4. Tracheostomaventil nach einem der Ansprüche 1 bis 3, **dadurch gekennzeichnet, dass** die Außendurchmesser der Dichtlamellen (4) in Richtung des Ventilkörpers (1) stetig zunehmen.

5. Tracheostomaventil nach einem der Ansprüche 1 bis 4, **dadurch gekennzeichnet, dass** die Dichtlamellen (4) an dem Kanülenstummel (2) angeformt sind.

6. Tracheostomaventil nach einem der Ansprüche 1 bis 5, **dadurch gekennzeichnet, dass** der Kanülenstummel (2) und die Dichtlamellen (4) aus Silikongummi bestehen.

7. Tracheostomaventil nach einem der Ansprüche 1 bis 6, **dadurch gekennzeichnet, dass** das Abschlussorgan (3) ein an das innere Ende des Kanülenstummels (2) angesetztes, zum Kanülenstummel (2) offenes Rohrstück (5) ist.

8. Tracheostomaventil nach Anspruch 7, **dadurch gekennzeichnet, dass** das Rohrstück (5) an der dem Kanülenstummel (2) gegenüberliegenden Wand eine Öffnung aufweist.

9. Tracheostomaventil nach Anspruch 7 oder 8, **dadurch gekennzeichnet, dass** das Rohrstück (5) auf der dem Kanülenstummel (2) gegenüberliegenden Seite vollständig geöffnet ist.

10. Tracheostomaventil nach einem der Ansprüche 7 bis 9, **dadurch gekennzeichnet, dass** an den Enden des Rohrstückes (5) jeweils ein Flansch (7) aus biegsamem Material angeformt ist, das in sich in Richtung Kanülenstummel (2) gebogen ist.

11. Tracheostomaventil nach Anspruch 10, **dadurch gekennzelch**net, dass der Kanülenstummel (2), die Dichtlamellen (4), das Rohrstück (5) und die Flansche (7) einstückig ausgebildet sind.

12. Tracheostomaventil mit einem Ventilkörper (1), der auf einer Tracheostomiekanüle (20) sitzt bzw. in dieser integriert ist, welche in ein Tracheostoma setzbar ist,
**gekennzeichnet durch**,
wenigstens zwei in Richtung der Hauptachse (H) des Einsatzstückes (21) der Tracheostomiekanüle (20) axial versetzte, auf dem Einsatzstück (21) umlaufende ringförmige Dichtlamellen (4), die sich nach Einsetzen der Tracheostomiekanüle (20) in das Tracheostoma (11) an die das Tracheostoma (11) umgebende Haut in dichtender Weise anlegen.

13. Tracheostomaventil nach Anspruch 12, **dadurch gekennzeichnet, dass** die Dichtlamellen (4) in Richtung auf den Ventilkörper (1) konvex ausgebildet sind.

14. Tracheostomaventil nach Anspruch 12 oder 13, **dadurch gekennzeichnet, dass** drei Dichtlamellen (4) vorgesehen sind.

15. Tracheostomaventil nach einem der Ansprüche 12 bis 14, **dadurch gekennzeichnet, dass** die Außendurchmesser der Dichtlamellen (4) in Richtung des Ventilkörpers (1) stetig zunehmen.

16. Tracheostomaventil nach einem der Ansprüche 12 bis 15, **dadurch gekennzeichnet, dass** die Dichtlamellen (4) an dem Einsatzstück (21) der Tracheostomiekanüle (20) angeformt sind.

17. Tracheostomaventil nach einem der Ansprüche 12 bis 16, **dadurch gekennzeichnet, dass** das Einsatzstück (21) der Tracheostomiekanüle (20) und die Dichtlamellen (4) aus Silikongummi bestehen.

## Claims

1. Tracheostoma valve having a valve body (1), which sits on a cannula stump (2), or is integrated in the latter, which can be placed in a tracheostoma and has a closure element (3) which can be introduced into the trachea, **characterised by** at least two annular sealing lamellae (4) axially offset in the direction of the main axis (H) of the cannula stump (2) and surrounding the cannula stump (2) and which, after inserting the cannula stump (2) into the tracheostoma (11), are placed against the skin surrounding the tracheostoma (11) in sealing manner.

2. Tracheostoma valve according to claim 1, **characterised in that** the sealing lamellae (4) are designed to be concave in the direction of the closure element (3).

3. Tracheostoma valve according to claim 1 or 2, **characterised in that** three sealing lamellae (4) are provided.

4. Tracheostoma valve according to one of claims 1 to 3, **characterised in that** the external diameters of the sealing lamellae (4) increase continuously in the direction of the valve body (1).

5. Tracheostoma valve according to one of claims 1 to 4, **characterised in that** the sealing lamellae (4) are moulded onto the cannula stump (2).

6. Tracheostoma valve according to one of claims 1 to 5, **characterised in that** the cannula stump (2) and the sealing lamellae (4) consist of silicone rubber.

7. Tracheostoma valve according to one of claims 1 to 6, **characterised in that** the closure element (3) is a piece of tube (5) which is attached to the inner end of the cannula stump (2) and open towards the cannula stump (2).

8. Tracheostoma valve according to claim 7, **characterised in that** the piece of tube (5) has an opening on the wall opposite the cannula stump (2).

9. Tracheostoma valve according to claim 7 or 8, **characterised in that** the piece of tube (5) is completely open on the side opposite the cannula stump (2).

10. Tracheostoma valve according to one of claims 7 to 9, **characterised in that** in each case a flange (7) made from flexible material, which is bent in itself in the direction of the cannula stump (2), is moulded onto the ends of the piece of tube (5).

11. Tracheostoma valve according to claim 10, **characterised in that** the cannula stump (2), the sealing lamellae (4), the piece of tube (5) and the flange (7) are designed to be integral.

12. Tracheostoma valve having a valve body (1), which sits on a tracheostomy cannula (20) or is integrated in the latter, which can be placed in a tracheostoma, **characterised by** at least two annular sealing lamellae (4) axially offset in the direction of the main axis (H) of the insertion piece (21) of the tracheostomy cannula (20) and surrounding the insertion piece (22) and which, after inserting the tracheostomy cannula (20) into the tracheostoma (11), are placed against the skin surrounding the tracheostoma (11) in sealing manner.

13. Tracheostoma valve according to claim 12, **characterised in that** the sealing lamellae (4) are designed to be convex in the direction of the valve body (1).

14. Tracheostoma valve according to claim 12 or 13, **characterised in that** three sealing lamellae (4) are provided.

15. Tracheostoma valve according to one of claims 12 to 14, **characterised in that** the external diameters of the sealing lamellae (4) increase continuously in the direction of the valve body (1).

16. Tracheostoma valve according to one of claims 12 to 15, **characterised in that** the sealing lamellae (4) are moulded onto the insertion piece (21) of the tracheostomy cannula (20).

17. Tracheostoma valve according to one of claims 12 to 16, **characterised in that** the insertion piece (21) of the tracheostomy cannula (20) and the sealing lamellae (4) consist of silicone rubber.

## Revendications

1. Valve de trachéostomie comportant un corps de valve (1), qui repose sur un bout de canule (2) ou est intégré dans celui-ci, lequel peut être posé dans une trachéostomie et comporte un organe de fermeture (3) pouvant être introduit dans la trachée,
**caractérisée par**
au moins deux lamelles d'étanchéité (4) annulaires périphériques, qui sont posées sur le bout de canule (2), qui sont décalées dans le sens axial dans la direction de l'axe principal (H) du bout de canule (2) et qui, après l'introduction du bout de canule (2) dans la trachéostomie (11), sont en appui étanche contre la peau entourant la trachéostomie (11).

2. Valve de trachéostomie selon la revendication 1, **caractérisée en ce que** les lamelles d'étanchéité (4) sont réalisées sous forme concave dans la direction vers l'organe de fermeture (3).

3. Valve de trachéostomie selon la revendication 1 ou 2, **caractérisée en ce que** trois lamelles d'étanchéité (4) sont prévues.

4. Valve de trachéostomie selon l'une quelconque des revendications 1 à 3, **caractérisée en ce que** les diamètres extérieurs des lamelles d'étanchéité (4) augmentent en continu vers le corps de valve (1).

5. Valve de trachéostomie selon l'une quelconque des revendications 1 à 4, **caractérisée en ce que** les lamelles d'étanchéité (4) sont formées sur le bout de canule (2).

6. Valve de trachéostomie selon l'une quelconque des revendications 1 à 5, **caractérisée en ce que** le bout de canule (2) et les lamelles d'étanchéité (4) sont réalisées en caoutchouc siliconé.

7. Valve de trachéostomie selon l'une quelconque des revendications 1 à 6, **caractérisée en ce que** l'organe de fermeture (3) est un bout de tube (5) posé sur l'extrémité intérieure du bout de canule (2) et ouvert vers le bout de canule (2).

8. Valve de trachéostomie selon la revendication 7, **caractérisée en ce que** le bout de tube (5) comporte une ouverture sur la paroi opposée au bout de canule (2).

9. Valve de trachéostomie selon la revendication 7 ou 8, **caractérisée en ce que** le bout de tube (5) est entièrement ouvert sur le côté opposé au bout de canule (2).

10. Valve de trachéostomie selon l'une quelconque des revendications 7 à 9, **caractérisée en ce qu'**une collerette (7) en matériau flexible est formée sur chacune des extrémités du bout de tube (5), laquelle est courbée en direction du bout de canule (2).

11. Valve de trachéostomie selon la revendication 10, **caractérisée en ce que** le bout de canule (2), les lamelles d'étanchéité (4), le bout de tube (5) et les collerettes (7) sont réalisés d'un seul tenant.

12. Valve de trachéostomie comportant un corps de valve (1), qui repose sur une canule de trachéostomie (20) ou est intégré dans celle-ci, laquelle peut être posée dans une trachéostomie,
**caractérisée par**
au moins deux lamelles d'étanchéité (4) annulaires périphériques, qui sont posées sur le bout d'introduction (21), qui sont décalées dans le sens axial dans la direction de l'axe principal (H) du bout d'introduction (21) et qui, après l'introduction de la canule de trachéostomie (20) dans la trachéostomie (11), sont en appui étanche contre la peau entourant la trachéostomie (11).

13. Valve de trachéostomie selon la revendication 12, **caractérisée en ce que** les lamelles d'étanchéité (4) sont réalisées sous forme convexe dans la direction vers le corps de valve (1).

14. Valve de trachéostomie selon la revendication 12 ou 13, **caractérisée en ce que** trois lamelles d'étanchéité (4) sont prévues.

15. Valve de trachéostomie selon l'une quelconque des revendications 12 à 14, **caractérisée en ce que** les diamètres extérieurs des lamelles d'étanchéité (4) augmentent en continu vers le corps de valve (1).

16. Valve de trachéostomie selon l'une quelconque des revendications 12 à 15, **caractérisée en ce que** les lamelles d'étanchéité (4) sont formées sur le bout d'introduction (21) de la canule de trachéostomie (20).

17. Valve de trachéostomie selon l'une quelconque des revendications 12 à 16, **caractérisée en ce que** le bout d'introduction (21) de la canule de trachéostomie (20) et les lamelles d'étanchéité (4) sont réalisées en caoutchouc siliconé.
